# EUROPEAN PATENT APPLICATION

(11) **EP 4 316 532 A1**
(43) Date of publication of application: **07.02.2024**
(21) Application number: 22779462.5
(22) Date of filing: 27.01.2022
(51) Int. Cl.: A61L 2/10, F21V 23/00, F21V 9/00, A61L 9/20

(54) **LIGHT IRRADIATION DEVICE**

(30) Priority: 31.03.2021 JP 2021061924
(71) Applicant: Daikin Industries, Ltd., Osaka-Shi, Osaka 530-0001 (JP)
(72) Inventor: SAITO, Tomoki, Osaka-shi, Osaka 530-0001 (JP); TANAKA, Toshio, Osaka-shi, Osaka 530-0001 (JP); OKUMOTO, Mamoru, Osaka-shi, Osaka 530-0001 (JP)
(74) Representative: Hoffmann Eitle
(86) International application number: PCT/JP2022/003012
(87) International publication number: WO 2022/209232

(57) **Abstract**

In response to a person touching a portion indicated by reference sign 1A on a desk 91 installed in a room R, for example, a control device 60 identifies this touched area as an identified area 80 that has been contaminated by the person. In this case, the control device 60 controls a drive mechanism 30 to direct a light source 20 to this identified area 80. The area touched by the person in the room R is thus UV-irradiated and sterilized.

## Description

### Technical Field

The present invention relates to a light irradiation device.

### Background Art

Patent Literature 1 discloses a UV LED irradiation system comprising: an odor sensor configured to output a contamination level signal indicating an odor level of a space; and a UV intensity determiner configured to determine an output state of a UV LED lighting device such that the intensity of ultraviolet light irradiation increases with an increase in odor level indicated by the contamination level signal.

### Citation List

### Patent Literature

Patent Literature 1: Japanese Patent Application Laid-Open Publication No. 2018-190604

### Summary of Invention

### Technical Problem

Areas subject to contamination and areas subject to events that may cause contamination are not fixed but may vary depending on the movement or conditions of a contamination source. Cleaning only certain predetermined areas may result in an incomplete cleaning with contamination remaining.

It is an object of the present invention to ensure more reliable performance of the cleaning that is performed to deal with contamination.

### Solution to Problem

A light irradiation device of the present disclosure comprises: an identification unit configured to identify an area indoors that has been contaminated by a person or an area indoors where an event that leads to possible contamination by a person has occurred; and an irradiation unit configured to move a light source for emitting ultraviolet (UV) light and/or change a direction of the light source to irradiate an identified area, which is an area identified by the identification unit, with UV light.

The use of this light irradiation device ensures more reliable performance of the cleaning that is performed to deal with contamination.

In some embodiments, the identification unit may be configured to identify an area touched by a person as an area that has been contaminated by the person. Thus, the area touched by the person can be UV-irradiated.

In some embodiments, the identification unit may be configured to identify an area in front of a face of a person who has coughed or sneezed as an area that has been contaminated by the person. Thus, the area in front of the face of the person who has coughed or sneezed can be UV-irradiated.

In some embodiments, the identification unit may be configured to identify an area that has been passed through by a person as an area that has been contaminated by the person. Thus, the area passed through by the person can be UV-irradiated.

In some embodiments, the identification unit may be configured to identify an area where a conversion between a plurality of persons has taken place as an area where an event that leads to possible contamination by the person has occurred. Thus, the area where a conversion between the plurality of persons has taken place can be UV-irradiated.

In some embodiments, the identification unit may be configured to identify an area where a conversation between a plurality of persons has taken place in which sound pressure of at least some of voices uttered during the conversation exceeds a predetermined threshold, as an area where an event that leads to possible contamination by the person has occurred. Thus, the area where a loud conversion has taken place can be UV-irradiated.

In some embodiments, the identification unit may be configured to identify an area where a person has uttered a voice without a mask as an area where an event that leads to possible contamination by the person has occurred. Thus, the area where the person uttered a voice without a mask can be UV-irradiated.

In some embodiments, the light source used by the irradiation unit for the UV irradiation may be provided on a mobile object, and the mobile object may autonomously move to the identified area to irradiate it with UV light. This facilitates UV irradiation of areas that are difficult to irradiate with UV light, as compared to when the light source is not provided on the mobile object.

In some embodiments, the mobile object may be configured to follow a person and irradiate with UV light the identified area that has been contaminated by the person and identified by the identification unit, or to follow a person and irradiate with UV light the identified area where an event that leads to possible contamination by the person has occurred due to the person and that has been identified by the identification unit. This allows for UV irradiation of the identified area while the mobile object is following the person.

In some embodiments, the identification unit may be configured to use UV light emitted from the irradiation unit to identify the contaminated area. Thus, the UV light emitted from the irradiation unit can be used to identify the contaminated area.

### Brief Description of the Drawings

FIG. 1 illustrates a light irradiation device.
FIG. 2 illustrates a hardware configuration of a control device.
FIG. 3 illustrates other conditions in a room.
FIGS. 4A and 4B are top-down views of the room.
FIG. 5 illustrates other conditions in the room.
FIG. 6 illustrates other conditions in the room.
FIG. 7 illustrates ultraviolet irradiation from a mobile object.
FIG. 8 illustrates another example process for ultraviolet irradiation from the mobile obj ect.

### Description of Embodiments

An exemplary embodiment will now be described with reference to the drawings.

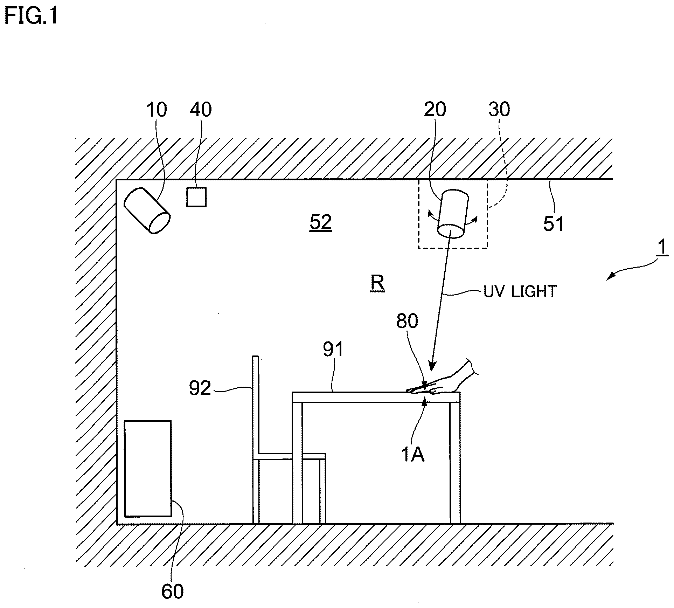

FIG. 1 illustrates a light irradiation device 1 according to the present embodiment.

The light irradiation device 1 of the present embodiment includes a camera 10 for capturing a video and a control device 60 for obtaining the video captured by the camera 10. The light irradiation device 1 further includes a light source 20 for emitting UV light and a drive mechanism 30 for changing the direction of the light source 20. The light irradiation device 1 further includes a microphone 40 for capturing sound.

The light irradiation device 1 is installed in a room R (indoors). Other equipment such as a desk 91 and a chair 92 is also installed in the room R.

In the present embodiment, the camera 10 and the control device 60 constitute the identification unit.

The identification unit identifies an area in the room R where contamination by a person has occurred. The identification unit also identifies an area in the room R where an event that leads to possible contamination by a person has occurred.

In the present embodiment, the light source 20, the drive mechanism 30, and the control device 60 constitute the irradiation unit. The irradiation unit directs the light source 20 to an identified area, which is an area identified by the above identification unit, and irradiates the identified area with UV light.

The light source 20 emits UV light in the wavelength range of 190 to 230 nm, for example. The use of UV light in the wavelength range of 190 to 230 nm can sterilize viruses and other substances adhering to the above identified area while minimizing the effects of the UV light on human skin and eyes.

The UV irradiation with such UV light in the wavelength range of 190 to 230 nm can be performed even if a person is present at the destination of the UV light.

The above wavelength range of 190 to 230 nm is not limiting; the UV light outside the wavelength range of 190 to 230 nm, such as at a wavelength of 254 nm, may also be used.

When using the UV light outside the wavelength range of 190 to 230 nm, a video captured by the camera 10 is used to determine whether there is any person at the destination of the UV light. The UV light is then emitted when there is no person at the destination of the UV light.

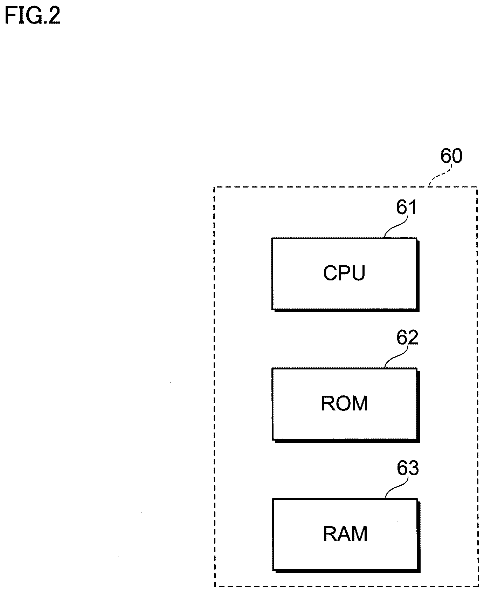

FIG. 2 illustrates a hardware configuration of the control device 60.

The control device 60 includes a central processing unit (CPU) 61 as an example of a processor, a read only memory (ROM) 62 storing control software and the like, and a random access memory (RAM) as a working area.

The CPU 61 may be a multi-core processor. The ROM 62 may be a rewritable, nonvolatile semiconductor memory. The control device 60 is a so-called computer.

Programs to be executed by the CPU 61 may be provided to the control device 60 in the form of a computer-readable recording medium such as a magnetic recording medium (magnetic tape, magnetic disk, etc.), optical recording medium (optical disk, etc.), magnetooptical recording medium, or a semiconductor memory.

Programs to be executed by the CPU 61 may also be provided to the control device 60 using communication means such as the Internet.

The control device 60, which functions as part of the identification unit, identifies, for example, an area touched by a person in the room R (see FIG. 1) as an identified area 80 that has been contaminated by the person.

Specifically, for example, in response to a person touching the portion indicated by reference sign 1A on the desk 91 installed in the room R, the control device 60 identifies this touched area as the identified area 80 contaminated by the person.

Specifically, the control device 60 analyzes a video, captured by the camera 10, at predetermined time intervals to identify areas touched by users.

In response to a person touching any area in the room R, the control device 60 identifies this touched area as the identified contaminated area 80.

In this case, the control device 60, which also functions as part of the irradiation unit, controls the drive mechanism 30 to direct the light source 20 to this identified area 80. The area touched by the person in the room R is thus UV-irradiated and sterilized.

While the example shown in FIG. 1 illustrates the light source 20 as being installed on a ceiling 51, the light source 20 may be installed on any other position such as on a sidewall 52, rather than on the ceiling 51.

In the present embodiment, the direction of the light source 20 is changed to change the destination of the UV light; alternatively, for example, the light source 20 may be moved to change the destination of the UV light.

Both of changing the direction of the light source 20 and moving the light source 20 may be performed to change the destination of the UV light.

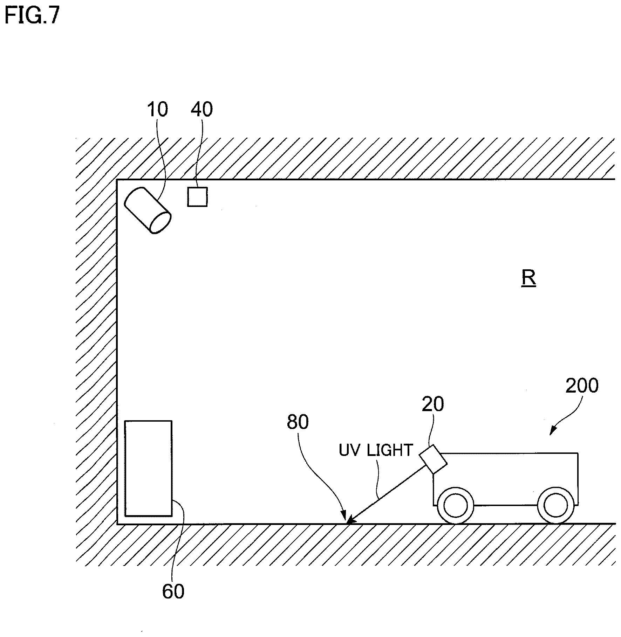

Alternatively, as described below, the light source 20 may be installed on a moving object 200 (see FIG. 7) that moves around the room R, and the moving object 200 may be moved to the identified area 80 to irradiate it with the UV light.

Still alternatively, multiple light sources 20 may be installed at mutually different positions. Such an arrangement allows the identified area 80 to be UV-irradiated from mutually different directions from the multiple light sources 20.

Such a multiple arrangement of the light sources 20 also reduces the situations where an obstacle is present between the light source 20 and the identified area 80 as compared to the use of a single light source 20, providing a greater assurance that the identified area 80 is UV-irradiated.

Other example areas that may be touched by a person include doorknobs, handrails, water faucets, and elevator buttons. Additional example areas include copy machines and the chair 92.

In the above example, the area touched by the person is identified based on the video from the camera 10. However, this is not limiting, and the area touched by the person may be identified based on output from any other sensor.

For example, a door may be equipped with an open/close detection sensor that detects opening/closing of this door, and in response to output from the open/close detection sensor signaling that the door has been opened, it may be determined that a person has touched the doorknob.

In this case, this doorknob is identified as the identified area 80 that has been contaminated, and thus UV-irradiated.

In another example, in response to output from a copy machine signaling that copying has started, it may be determined that a person has touched the start button for copying.

In this case, this start button is identified as the identified area 80 that has been contaminated, and thus UV-irradiated.

Additionally, the control device 60 of the present embodiment identifies an area in front of the face of a person who has just coughed or sneezed as the identified area 80 that has been contaminated by the person.

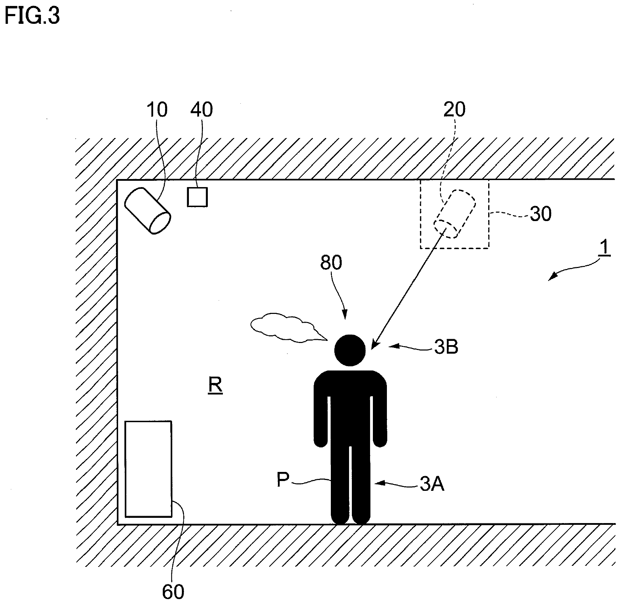

Specifically, for example, in response to a person P indicated by reference sign 3A in FIG. 3 (which shows other conditions in the room R) coughing or sneezing, the control device 60 identifies the area indicated by reference sign 3B as the identified area 80 that has been contaminated by the person.

In this case, the control device 60 controls the drive mechanism 30 to direct the light source 20 to this identified area 80 in front of the face of this person P. Thus, this area, where saliva and other particles from the person's mouth have been scattered, is UV-irradiated.

Additionally, the control device 60 of the present embodiment identifies an area passed through by a person P as the identified area 80 that has been contaminated by the person P.

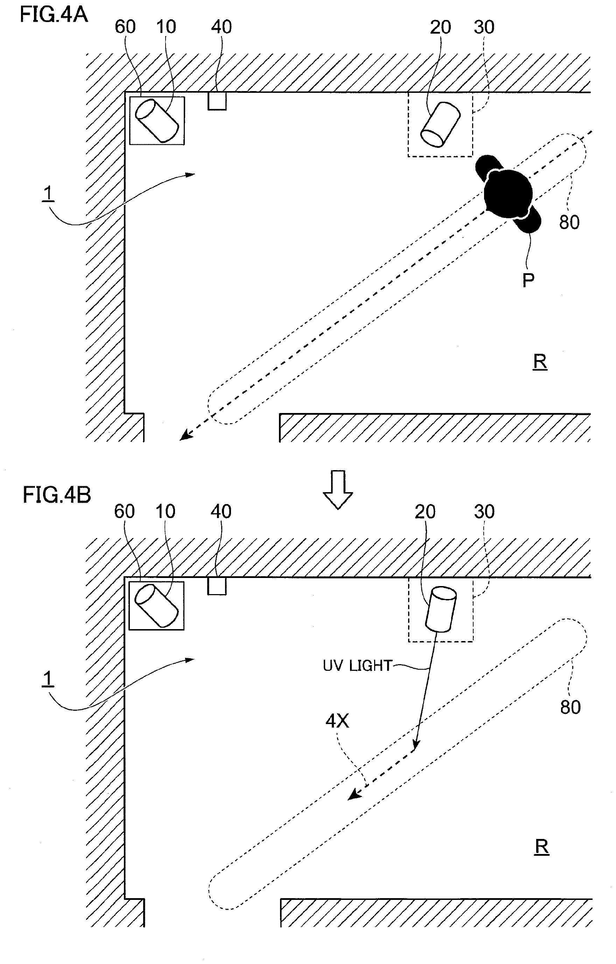

FIGS. 4A and 4B are top-down views of the room R.

In the present embodiment, in response to a person P passing through a room R as shown in FIG. 4A, the control device 60 identifies the area passed through by this person P as the identified area 80 that has been contaminated by the person P.

In this case, the control device 60 controls the drive mechanism 30 to sequentially irradiate this identified area 80 with UV light as shown in FIG. 4B.

More specifically, the control device 60 controls the drive mechanism 30 for the light source 20 such that the destination of the UV light moves along the identified contaminated area 80, as indicated by arrow 4X in FIG. 4B.

In other words, the control device 60 controls the drive mechanism 30 for the light source 20 such that the destination of the UV light moves along the area passed through by the person P.

Thus, the UV irradiation is performed along the path in the room R taken by the person P.

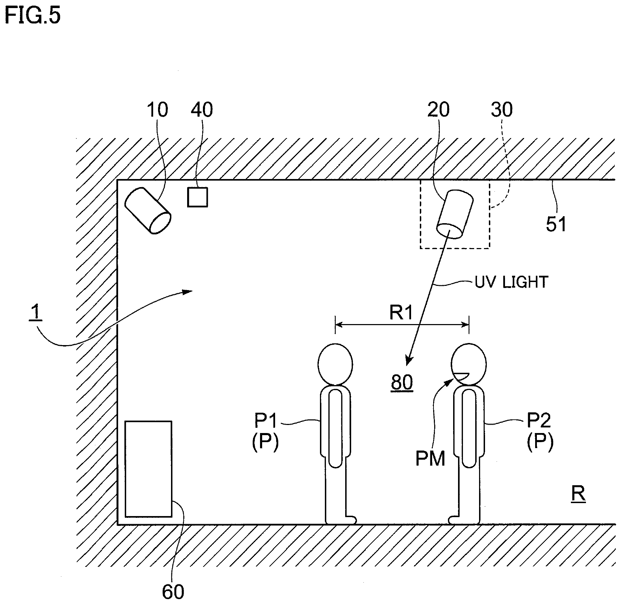

FIG. 5 illustrates other conditions in the room R. In FIG. 5, the room R is shown in a side view.

The control device 60 further identifies an area where a conversation between a plurality of persons P has taken place as the identified area 80 where an event that leads to possible contamination by the person(s) P has occurred.

Specifically, the control device 60 analyzes the video captured by the camera 10 to determine whether a conversation between a plurality of persons P has taken place.

Upon determining that a conversation between a plurality of persons P has taken place, the control device 60 identifies the area where the conversation between the plurality of persons P has taken place as the identified area 80 where an event that leads to possible contamination by the person(s) P has occurred.

In the conditions shown in FIG. 5, there is a plurality of persons P in the room R. One person P1 and another person P2 included in this plurality of persons P are standing at a distance R1 that is less than a predetermined threshold. Additionally, in the conditions shown in FIG. 5, the mouth of at least one of the one person P1 and the other person P2 is moving. Specifically, in this example, the mouth PM of the other person P2 is moving.

If the video captured by the camera 10 indicate such conditions shown in FIG. 5, the control device 60 determines that a conversation has taken place between the plurality of persons P.

In this case, the control device 60 identifies the area where this conversation has taken place as the identified area 80 where an event that leads to possible contamination by the person(s) P has occurred.

In this case, the control device 60 causes the identified area 80 to be UV-irradiated, in the same manner as described above, after the plurality of persons P leaves the identified area 80. Thus, the area where the conversation took place is UV-irradiated.

In the above example, the UV irradiation is performed after the plurality of persons P leaves the area 80. However, if UV light in the wavelength range of 190 to 230 nm as described above is used, the UV irradiation may be performed while the plurality of persons P are present.

In the above example, the control device 60 determines the occurrence of a conversation between the plurality of persons P based on the video captured by the camera 10. However, the control device 60 may make this determination by further taking into account the output from the microphone 40 installed in the room R

Specifically, the control device 60 may determine the occurrence of a conversation between the plurality of persons P based on, for example, the distance R1 between the one person P1 and the other person P2 and the output from the microphone 40.

In this case, for example, if the distance R1 between the one person P1 and the other person P2 is less than a predetermined threshold and the output from the microphone 40 indicates the issuing of an utterance, the control device 60 determines the occurrence of a conversation between the plurality of persons.

In another implementation, the control device 60 may provide the UV irradiation of the identified area 80, where the conversation has taken place, only when voices during the conversation are loud, rather than consistently providing the UV irradiation.

Specifically, for such a process, the control device 60 determines whether sound pressure of at least some of the voices uttered during any conversation between a plurality of persons P exceeds a predetermined threshold.

More specifically, the control device 60 obtains information from the microphone 40 installed in the room R to recognize the sound pressure of the conversation and determine where the sound pressure of at least some of the voices uttered during the conversation exceeds the predetermined threshold.

In response to the sound pressure of some of the voices exceeding the predetermined threshold, the control device 60 identifies an area where this conversation has taken place as the identified area 80 where an event that leads to possible contamination by the person(s) P has occurred. Thus, the control device 60 causes this identified area 80 to be UV-irradiated in the same manner as described above.

In this implementation, the UV irradiation is performed only when a loud conversation has taken place.

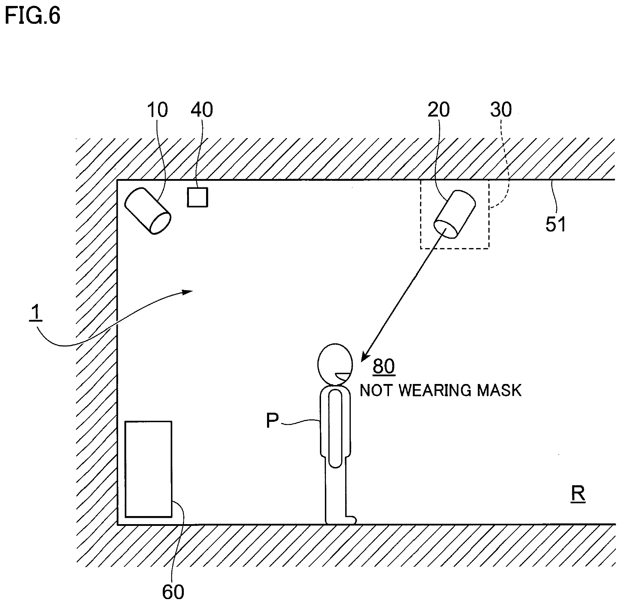

FIG. 6 illustrates other conditions in the room R. In FIG. 6, the room R is shown in a side view.

The control device 60 analyzes the video captured by the camera 10 to determine whether a person P in the room R is wearing a mask.

In response to the person P uttering a voice without a mask, the control device 60 identifies an area where this person P has uttered the voice as the identified area 80 where an event that leads to possible contamination by the person P has occurred.

In this case, the control device 60 controls the drive mechanism 30 to irradiate this identified area 80 with UV light, in the same manner as described above. Thus, the area where the person has uttered the voice without a mask is UV-irradiated.

Additionally, in response to a person P coughing or sneezing without a mask, the control device 60 identifies an area where this person P has coughed or sneezed as the identified area 80.

In this case, too, the control device 60 controls the drive mechanism 30 to irradiate this identified area 80 with UV light, in the same manner as described above. Thus, the area where the person P has coughed or sneezed without a mask is UV-irradiated.

Additionally, the control device 60 may identify an area where a plurality of persons P has had a meal as the identified area 80.

In this case, the control device 60 determines that a plurality of persons is having a meal if one person P and another person P are at a distance that is less than a predetermined threshold and food is present in front of each person P.

The control device 60 then identifies this area where the meal is being taken as the identified area 80. Thus, this identified area 80 is UV-irradiated in the same manner as described above.

FIG. 7 illustrates UV irradiation from a mobile object 200. In FIG. 7, the room R is shown in a side view.

In the example configuration shown in FIG. 7, the mobile object 200 is equipped with the light source 20. The mobile object 200 is further equipped with known mechanisms for moving the mobile object 200, such as motors and wheels.

In this configuration example, the identified area 80 that has been contaminated and/or the identified area 80 where an event that may cause contamination has occurred are UV-irradiated as this mobile object 200 moves.

In this example, the control device 60 identifies the identified area 80. The location information indicating the position of this identified area 80 is then transmitted from the control device 60 to the mobile object 200.

Upon receiving this location information, the mobile object 200 autonomously moves to the identified area 80 based on this location information and irradiates the identified area 80 with UV light.

In this example, the identified area 80 is identified by the control device 60 provided separately from the mobile object 200.

However, such an implementation is not limiting, and the mobile object 200 may be equipped with features for identifying the identified area 80, such as the camera 10 and the control device 60, so that the mobile object 200 can identify the identified area 80 by itself.

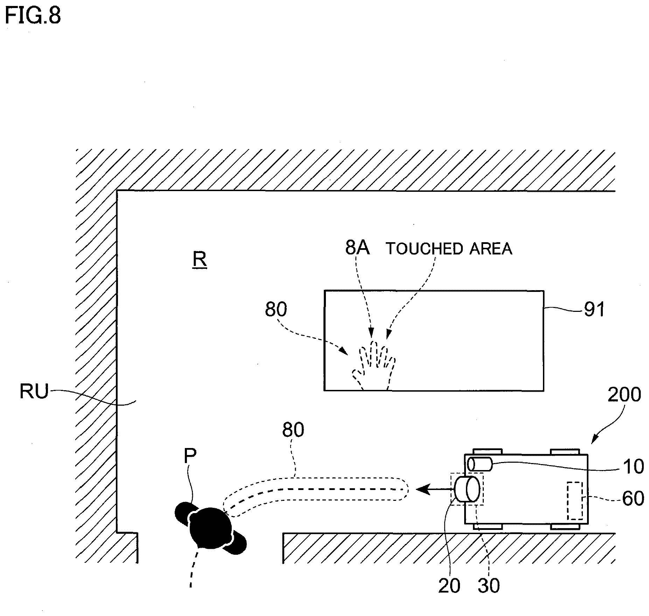

FIG. 8 illustrates another example process for UV irradiation from the mobile object 200. In FIG. 8, the room R is shown in a top-down view.

In this processing example, the camera 10 is provided on the mobile object 200, and the control device 60 provided on the mobile object 200 identifies the identified area 80.

In this processing example, the control device 60 analyzes the video captured by the camera 10 to detect a person P in the room R. In this processing example, the mobile object 200 follows the person P as he/she moves around, as shown in FIG. 8.

In this processing example, the control device 60 provided on the mobile object 200 identifies an area passed through by this person P as the identified area 80. The control device 60 then controls the drive mechanism 30 such that the light source 20 faces forward and downward at an angle for UV irradiation of the area passed through by the person P.

In this implementation, the UV irradiation of the floor surface RU from the light source 20 on the mobile object 200 following the person P is performed while the mobile object 200 moves. In this manner, the UV irradiation is performed along the path taken by the person.

In this processing example, the control device 60 provided on the mobile object 200 also identifies any areas touched by the person P who is the target of the tracking, based on the video captured by the camera 10.

In other words, the control device 60 analyzes the video captured by the camera 10 to identify not only the area passed through by the person P but also the identified area 80 (see reference sign 8A) such as the area touched by the person P.

Upon identifying this identified area 80, the control device 60 controls the drive mechanism 30 to direct the light source 20 on the mobile object 200 to this identified area 80 (identified area 80 indicated by reference sign 8A) and irradiate it with UV light.

The mobile object 200 may be equipped with a special light source 20 for UV irradiation of this identified area 80, separately from the light source 20 for UV irradiation of the path taken by the person, and the mobile object 200 may use this special light source 20 to irradiate this identified area 80 with UV light.

Additionally, the mobile object 200 also irradiates with UV light an area where a conversation has taken place between the person P who is the target of tracking and another person.

In other words, in response to an event that may cause contamination occurring due to the person P who is the target of the tracking, the mobile object 200 identifies the area where this event has occurred as the identified area 80. Thus, the mobile object 200 also irradiates this identified area 80 with UV light.

In another example processing, for example, the mobile object 200 may be moved randomly in the room R, independently of any person P in the room R.

In such an implementation, the mobile object 200 irradiates with UV light surrounding objects, such as the floor RU and doorknobs, and uses the camera 10 to capture a video of the UV-irradiated areas.

The control device 60 on the mobile object 200 then analyzes the captured video to determine where there are any contaminated areas. In other words, the control device 60 determines whether there is contamination.

When a contaminated floor RU or doorknob is UV-irradiated, the UV light acts as a black light and causes the contaminated area to emit light. This allows for determining the presence or absence of contamination. The control device 60 analyzes the video captured by the camera 10 to determine whether such luminescence is present.

If there is any contaminated area, the control device 60 controls the drive mechanism 30 to further irradiate this area with UV light. Thus, again, the identified area 80 can be UV-irradiated.

In the above example, a special mobile object 200 for UV sterilization has been described. However, each of the above functions for UV sterilization may be provided on a mobile object 200 such as a cleaning robot.

Also, the mobile object 200 is not limited to those that move along the floor, and may include those that move through the air, such as drones.

Still alternatively, for example, the mobile object 200 may be one that can interact with people, such as a communication robot, and such a mobile object 200 may be provided with the above functions for UV sterilization.

The embodiments described above can be viewed as including the following aspects.
(1) Alight irradiation device 1 comprises: an identification unit configured to identify an area indoors that has been contaminated by a person P or an area indoors where an event that leads to possible contamination by a person P has occurred; and an irradiation unit configured to move a light source 20 for emitting ultraviolet light and/or change a direction of the light source 20 to irradiate an identified area 80, which is an area identified by the identification unit, with UV light.
   The use of the light irradiation device 1 ensures more reliable performance of the cleaning that is performed to deal with contamination.
(2) The identification unit may be configured to identify an area touched by a person P as an area that has been contaminated by the person P. Thus, the area touched by the person P can be UV-irradiated.
(3) The identification unit may be configured to identify an area in front of a face of a person P who has coughed or sneezed as an area that has been contaminated by the person P. Thus, the area in front of the face of the person P who has coughed or sneezed can be UV-irradiated.
(4) The identification unit may be configured to identify an area that has been passed through by a person P as an area that has been contaminated by the person P. Thus, the area passed through by the person P can be UV-irradiated.
(5) The identification unit may be configured to identify an area where a conversion between a plurality of persons P has taken place as an area where an event that leads to possible contamination by the person P has occurred. Thus, the area where a conversion between the plurality of persons P has taken place can be UV-irradiated.
(6) The identification unit may be configured to identify an area where a conversation between a plurality of persons P has taken place in which sound pressure of at least some of voices uttered during the conversation exceeds a predetermined threshold, as an area where an event that leads to possible contamination by the person P has occurred. Thus, the area where a loud conversion has taken place can be UV-irradiated.
(7) The identification unit may be configured to identify an area where a person P has uttered a voice without a mask as an area where an event that leads to possible contamination by the person P has occurred. Thus, the area where the person P uttered a voice without a mask can be UV-irradiated.
(8) The light source 20 used by the irradiation unit for UV irradiation may be provided on the mobile object 200, and the mobile object 200 may autonomously move to the identified area 80 to irradiate it with UV light. This facilitates UV irradiation of areas that are difficult to irradiate with UV light, as compared to when the light source 20 is not provided on the mobile object 200.
(9) The mobile object 200 may be configured to follow a person P and irradiate with UV light the identified area 80 that has been contaminated by the person P and identified by the identification unit, or to follow a person P and irradiate with UV light the identified area 80 where an event that leads to possible contamination by the person P has occurred due to the person P and that has been identified by the identification unit. This allows for UV irradiation of the identified area 80 while the mobile object 200 is following the person P.
(10) The identification unit may be configured to use UV light emitted from the irradiation unit to identify the contaminated area. Thus, the UV light emitted from the irradiation unit can be used to identify the contaminated area.

It will be understood that the configurations described above are not limited to the above embodiments and variations thereof and can be modified without departing from the spirit of the present disclosure. In other words, it will be understood that various modifications can be made to the forms and details without departing from the sprit and scope of the appended claims.

For example, part of the configurations described above may be omitted, or other functions may be added to the configurations described above.

While multiple configuration examples have been described above, a configuration included in one configuration example may be replaced with a configuration included in another configuration example, or a configuration included in one configuration example may be added to another configuration example.

### Reference Signs List

- 1: Light irradiation device
- 10: Camera
- 20: Light source
- 30: Drive mechanism
- 40: Microphone
- 60: Control device
- 80: Identified area
- 91: Desk
- 92: Chair
- 200: Mobile object
- P: Person
- R: Room

## Claims

1. Alight irradiation device comprising:
an identification unit configured to identify an area indoors that has been contaminated by a person or an area indoors where an event that leads to possible contamination by a person has occurred; and
an irradiation unit configured to move a light source for emitting ultraviolet light and/or change a direction of the light source to irradiate an identified area, which is an area identified by the identification unit, with ultraviolet light.

2. The light irradiation device according to claim 1, wherein the identification unit is configured to identify an area touched by a person as an area that has been contaminated by the person.

3. The light irradiation device according to claim 1, wherein the identification unit is configured to identify an area in front of a face of a person who has coughed or sneezed as an area that has been contaminated by the person.

4. The light irradiation device according to claim 1, wherein the identification unit is configured to identify an area that has been passed through by a person as an area that has been contaminated by the person.

5. The light irradiation device according to claim 1, wherein the identification unit is configured to identify an area where a conversion between a plurality of persons has taken place as an area where an event that leads to possible contamination by the person has occurred.

6. The light irradiation device according to claim 5, wherein the identification unit is configured to identify an area where a conversation between a plurality of persons has taken place in which sound pressure of at least some of voices uttered during the conversation exceeds a predetermined threshold, as an area where an event that leads to possible contamination by the person has occurred.

7. The light irradiation device according to claim 1, wherein the identification unit is configured to identify an area where a person has uttered a voice without a mask as an area where an event that leads to possible contamination by the person has occurred.

8. The light irradiation device according to claim 1, wherein a light source used by the irradiation unit for the ultraviolet light irradiation is provided on a mobile object, and
the mobile object is configured to move autonomously to the identified area and irradiate the identified area with ultraviolet light.

9. The light irradiation device according to claim 8, wherein the mobile object is configured to follow a person and irradiate with ultraviolet light the identified area that has been contaminated by the person and identified by the identification unit, or to follow a person and irradiate with ultraviolet light the identified area where an event that leads possible contamination by the person has occurred due to the person and that has been identified by the identification unit.

10. The light irradiation device according to claim 1, wherein the identification unit is configured to use ultraviolet light emitted from the irradiation unit to identify the contaminated area.
